(19) Europäisches Patentamt
European Patent Office
Office européen des brevets

(11) Publication number: **0 008 854**
**B1**

(12) **EUROPEAN PATENT SPECIFICATION**

(45) Date of publication of patent specification: **09.03.83**

(51) Int. Cl.³: **C 07 D 239/18,**
A 01 N 43/54

(21) Application number: **79301312.9**

(22) Date of filing: **06.07.79**

(54) **Pentadienone hydrazone, and its use as insecticidal agent.**

(30) Priority: **17.08.78 US 934433**
**17.08.78 US 934441**

(43) Date of publication of application:
**19.03.80 Bulletin 80/6**

(45) Publication of the grant of the patent:
**09.03.83 Bulletin 83/10**

(84) Designated Contracting States:
**CH DE FR GB IT**

(56) References cited:
**US - A - 3 878 201 (A. S. TOMCUFCIK)**
*** claim 1 ***
**US - A - 4 087 525 (J. B. LOVELL)**
*** claims 1, 4 ***
**US - A - 3 726 920 (D. W. HENRY)**
**US - A - 3 931 152 (A. S. TOMCUFCIK et al.)**

**The file contains technical information submitted after the application was filed and not included in this specification**

(73) Proprietor: **AMERICAN CYANAMID COMPANY**
**Berdan Avenue**
**Wayne New Jersey 06904 (US)**

(72) Inventor: **Drabb jr., Thomas Walter**
**918 Chestnut Avenue**
**Trenton, New Jersey (US)**
Inventor: **Lovell, James Byron**
**Box 347-B, RR 1, Woonsamonsa Road**
**Pennington New Jersey (US)**

(74) Representative: **Allam, Peter Clerk et al,**
**LLOYD WISE, TREGEAR & CO. Norman House**
**105-109 Strand**
**London WC2R 0AE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

Pentadienone hydrazone and its use as insecticidal agent

This invention relates to a novel pentadienone hydrazone useful as an insecticidal agent.

U.S. Patent No. 4,087,525 discloses the use as insecticidal agents of pentadienone hydrazones of the formula:

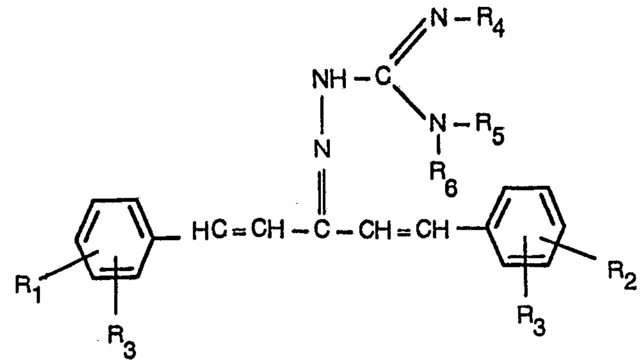

wherein $R_1$ and $R_2$ each represent hydrogen, halogen, $CF_3$, $C_1$—$C_4$ alkyl, $C_1$—$C_4$ alkoxy or $C_1$—$C_4$ alkylthio; $R_3$ is hydrogen or methyl, provided that when $R_3$ is methyl, $R_1$ and $R_2$ are also methyl, $R_4$ and $R_5$ represent hydrogen or $C_1$—$C_4$ alkyl, and when taken together, an alkylene group of 2 to 6 carbon atoms, methyl or phenyl alkylene group of 2 to 4 carbon atoms, a dimethyl alkylene group of 2 to 4 carbon atoms or 1,2-cyclohexylene; and $R_6$ is hydrogen or $C_1$—$C_4$ alkyl; and salts thereof.

The pentadienone hydrazone of the present invention is structurally related to compounds of U.S. Patent No. 4,087,525, but is unexpectedly found to be superior as an insecticide as compared with its close analogs taught in this prior art.

The compound of the present invention is 1,5-bis[*p*-(trifluoromethoxy)-phenyl]-1,4-pentadien-3-one-(1,4,5,6-tetrahydro-5,5-dimethyl-2-pyrimidinyl)hydrazone, and its inorganic acid addition salts.

The compounds of the invention, in its free base form, may be represented by the structural formula:

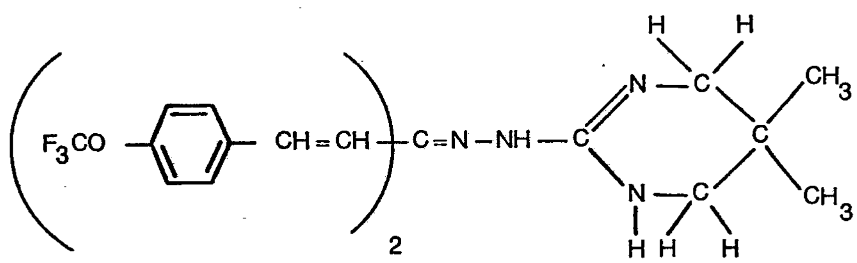

(I)

The compound of this invention can be conveniently prepared by a reaction sequence graphically illustrated and described as follows:

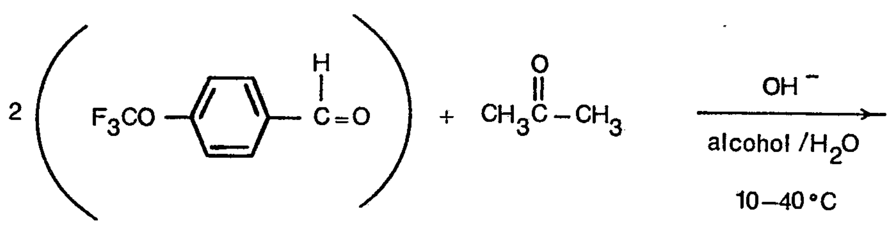


*p*-Trifluoromethoxybenzaldehyde

(II)

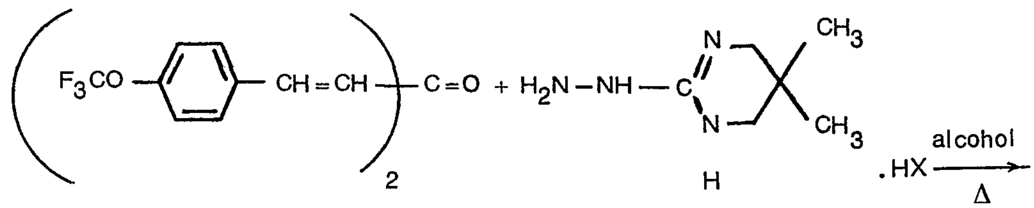


(I)

(III)
1,5-Bis[*p*-trifluoromethoxy)-phenyl]-1,4-pentadien-3-one

(IV)
(1,4,5,6-tetrahydro-5-5-dimethyl-2-pyrimidinyl)-hydrazine

wherein HX represents a mono or divalent inorganic acid, such as hydrochloric, hydrobromic or hydriodic acid. In the above sequence, the desired product (I) is shown as the HX salt. The corresponding pentadienone hydrazone free base may be obtained by treating the HX salt with an aqueous solution of a base, such as sodium or potassium bicarbonate, sodium or potassium carbonate, sodium or potassium hydroxide or ammonium hydroxide.

As above illustrated, two moles of *p*-trifluoromethoxybenzaldehyde (II) are reacted with one mole of acetone in an aqueous alcohol in the presence of an alkali metal base such as sodium or potassium hydroxide at a temperature range of 10 to 40°C, preferably 20 to 25°C for a period of time from one to three hours, or until the condensation reaction is essentially complete to yield the corresponding pentadienone (III). Next, the so-obtained pentadienone is condensed with an equimolar (or, if desired, slight excess) amount of a salt of (1,4,5,6-tetrahydro-5,5-dimethyl-2-pyrimidinyl)hydrazine (IV) in an alcohol at 50°C to 80°C or at the boiling point of said alcohol for a period of time of one to five hours or until the condensation reaction is essentially complete to yield the desired pentadienone hydrazone (I) in the form of its HX salt. Exemplary of the alcohols used in the above reaction sequences are methanol, ethanol, isopropanol, and mixtures thereof. The pentadienone hydrazone may be recovered from the above salt, if desired, by treating said salt with a dilute aqueous solution of an inorganic base, such as sodium or potassium bicarbonate, sodium or potassium carbonate, sodium or potassium hydroxide, or ammonium hydroxide.

The compound of the present invention finds utility in the control of insects and ants, Family Formicidae, by contacting said insects with, and/or applying to their habitat (but not a human or animal body) or food supply, an insecticidally effective amount of the compound. Further, the compound finds utility in protecting agronomic crops, trees, shrubs and ornamentals from attack by insects, by applying to the crops an insecticidally effective amount of the compound. In practice, generally 0.14 kg-hectare to 11.2 kg/hectare, and preferably 0.56 kg/hectare to 4.48 kg/hectare of the compound is effective for insect control other than through the use of baits as disclosed below and/or for crop protection.

The compound of this invention can be applied in either liquid or solid form. It may be applied in solid form as dusts or dust concentrates, or in liquid form as emulsifiable concentrates, flowable (thixotropic) formulations or wettable powders which are dispersed in water or other inexpensive liquid for application as a finely divided spray.

The present compound may also be prepared in the form of an attractant bait which is distributed to the locus or habitat of the insects sought to be controlled.

A typical emulsifiable concentrate can be prepared by admixing from 12% to 29% by weight of the present compound, 8% to 12% by weight of a blend of nonionic emulsifiers such as T—MULZ 339 (sold by Thompson-Hayward of Kansas City, Kansas), or polyoxyethylene derivatives and blends with alkyl aryl sulfonates, and 59% to 80% by weight of cyclohexanone or a heavy aromatic solvent having a mixed aniline point between —1°C and 35.0°C, a specific gravity between 0.880 and 1.5 at 15.5°C and an aromatic content of 60% to 100%. These formulations provide from 119.0 g/liter to 239.6 g/liter of active compound and are generally diluted with water for application as a dilute liquid. However, said formulations can also be applied in the form of undiluted discrete droplets as low volume or ultra-low volume sprays. For such application, the emulsifiable concentrate is usually applied with apparatus designed to disperse the liquid in the form of finely divided discrete droplets having a mass median diameter of from 25 to 150 microns ($25x10^{-6}$ and $150x10^{-6}$ meters).

A typical wettable powder formulaion can be prepared by grinding together about 34% by weight of a synthetic calcium silicate, 12% by weight of a dispersing agent such as sodium lignosulfonate, 4% by weight of a wetting agent such as an alkyl aryl sulfonate, and 50% by weight of the active compound. Such formulation is generally dispersed in water for application as a liquid spray.

It has been found that the present pentadienone hydrazone is useful for the control of insects especially a wide variety of Lepidopterous insects.

The compound of this invention, moreover, is active against Lepidopterous larvae, such as southern armyworms [*Spodoptera eridania* (Cramer)], cabbage loopers [*Trichoplusia ni* (Hübner)], and tobacco budworms [*Heliothis virescens* (Fabricius)], at 10 to 1000 ppm rates. It does not appear to be

especially toxic to most beneficial insects and thus is useful for pest management and integrated control programs. Moreover, the compound shows virtually no phytotoxicity to plants at rates of application up to 11.2 kg/hectare.

Advantageously, the pentadienone hydrazone compound of the present invention is active as a stomach poison. Thus, it is effective against insects with chewing mouth parts (Orthopterous insects such as cockroaches, grasshoppers, crickets and Isopterous insects such as termites) as well as those with sponge and lapping mouth parts (Dipterous insects such as flies). It is effective for the control of fire ants, such as the southern fire ant, *Solenopsis xyloni,* the black imported fire ant, *Solenopsis richteri,* and the red imported fire ant, *Solenopsis invicta.* It is also effective for the control of ants such as the big-headed ant, *Pheidole magecephala*, and the Argintine ant, *Irdomyrmax humulis*, that are dominate pests in pineapple and sugarcane fields, and for the control of many species of ants that are classified under the general category of household ants. Ants are serious economic and public health pests. Serious problems created by fire ants include stinging of humans and livestock, feeding on plants, particularly on seedlings and on germinating seeds, damage to farm machinery that strike ant mounds, loss of crops and refusal of workers to enter infested fields to cultivate and harvest crops. Ants invade houses, crawl over food, carry bits of food to their nests and also cause damage by establishing their nests in the woodwork of houses and other wooden buildings.

Control of these pests can be achieved with treated baits that are distributed in or adjacent to the infested area, such as pasture, park dwellings or other locations in which ant control is desired, and made available to worker ants. The workers carry the treated bait to the colony where it is consumed by the queens and the young ants, leading to their destruction.

In practice, generally from 1.25 g/ha to 75.0 g/ha, and preferably from 2.5 g/ha to 37.5 g/ha of the present pentadienone hydrazone is effective for fire ant control and/or for crop protection from ants and 0.0625% to 4% by weight, and preferably 0.125% to 2.0% by weight of the present pentadienone hydrazone is effective for the control of house ants and/or insects that are controlled by bait.

Baits can be prepared, for example, by admixing said formula (I) compounds with peanut butter or citrus pulp, vegetable oils such as soybean oil, animal fats such as lard and tallow, and with or without an organic filler such as bran, and/or an attractant such as lecithin. The composition is then placed in soda straws or on a carrier such as puffed grain, corncob grits and/or starch matrix and distributed in the area of the colony or infestation. Use of these baits has particular advantage, since such method of distribution poses little or no hazard to nontarget organisms that may frequent the infested area.

The invention is further illustrated by the examples set forth below which are provided only by way of illustration and are not deemed to be limiting thereof. Unless otherwise noted, all parts are by weight.

Example 1

Preparation of p-Trifluoromethoxybenzaldehyde.

To a solution of hexamethylenetetramine (300 g; 2.14 mole) in ethanol (900 ml) and water (600 ml) a mixture of $\alpha$-bromo-4-trifluoromethoxytoluene and $\alpha,\alpha$-dibromo-4-trifluoromethoxytoluene (300 g; 1.11 mole; 76:24 mixture) is added. The reaction mixture is stirred and heated at reflux for 3 hours under a nitrogen atmosphere, and then stirred overnight at room temperature.

From the above reaction mixture a cloudy liquid (144.5 g) is isolated and then distilled under vacuum to afford 122.2 g (58%) of title product, a colorless liquid, b.p. 66—74°C at 5—7 mm. NMR (in $CCl_4$) [$\delta = 6.15$ and 6.75 (AB doublets, 4H, J = 8Hz, aromatics), 8.85 (S, 1H, —CHO)]. IR. 1710 $cm^{-1}$ (carbonyl stretch).

The required mixture of $\alpha$-bromo- and $\alpha,\alpha$-dibromo-4-trifluoromethoxytoluene is obtained through the bromination of *p*-trifluoromethoxytoluene by the method of Example 8.

Example 2

Preparation of 1,5-Bis[p-(trifluoromethoxy)phenyl]-1,4-pentadien-3-one.

Acetone (8.7 g; 0.15 mole) is added to a solution of *p*-trifluoromethoxybenzaldehyde (57.0 g; 0.30 mole) in methanol (200 ml). The solution is cooled to 20°C and 10% aqueous sodium hydroxide (8 ml) is added dropwise in about 5 minutes. The reaction mixture is then stirred at room temperature for 1.5 to 2 hours. During this period a yellow solid precipitates from the solution. The mixture is chilled overnight, filtered and the isolated yellow solid dried to afford 24.8 g (41%) of title product, m.p. 121—123°C. NMR (acetone-d6) [$\delta$ = 7.0—8.0 (M, 12H, aromatics and vinyl)]; IR 1590—1650 $cm^{-1}$ (several bands).

Example 3

Preparation of 1,5-Bis[*p*-(trifluoromethoxy)phenyl]-1,4-pentadien-3-one-(1,4,5,6-tetrahydro-5,5-dimethyl-2-pyrimidinyl)hydrazone

A mixture of 1,5-bis[*p*-(trifluoromethoxy)phenyl]-1,4-pentadien-4-one (4.02 g; 0.01 mole), (1,4,5,6-tetrahydro-5,5-dimethyl-2-pyrimidinyl)hydrazine hydroiodide (2.70 g; 0.01 mole) and ethanol (25 ml) is stirred and heated at reflux for 3 hours. The ethanol is then stripped from the reaction mixture and the residue washed thoroughly with ether. The residue is then stirred with ether (100 ml) and

saturated sodium carbonate solution (50 ml). After about 15 minutes the ether layer is separated, dried and evaporated. The residual yellow solid is recrystallized from a benzene-hexane mixture to afford 2.6 g (50%) of title product, a yellow solid, m.p. 170—172°C.

Analysis calculated for $C_{25}H_{24}O_2N_4F_6$: C 57.05; H 4.56; N 10.65;
Found: C 56.42; H 4.59; N 10.46.

Example 4

Evaluation of the Insecticidal Activity of the Compound of the Invention

The activity of the compound of this invention is demonstrated by the following tests, wherein the pentadienone hydrazone is evaluated against test insect species at rates ranging from 10 to 1000 ppm. Test formulations and procedures used for evaluation are as follows:

*Test Formulations*

A. 100 Mg of the test material is weighed, placed in a funnel over a 113 g narrow-mouth bottle, and rinsed into the bottle with a 35 ml scoop of acetone, followed by a scoop of water and another scoop of acetone to yield 1000 ppm in 65% acetone. If the material is not soluble, it is broken up with a glass rod and used as a suspension.

B This stock solution ("A") is used to make 300 ppm solutions or suspensions by pipetting 30 ml of "A" into a bottle containing 70 ml of 50% acetone to yield 300 ppm. Further dilutions in 50% acetone are made as required.

C. Tests requiring 10 ppm in acetone solutions: 1 ml of "A" is pipetted into 99 ml of acetone to yield 10 ppm. Additional dilutions are made using 50% acetone as required.

*Initial Tests*

Tobacco Budworm — *Heliothis virescens* (Fabricius).

A cotton plant with 2 true leaves expanded is dipped for 3 seconds with agitation in 300 ppm solution. A 1.27 to 1.91 cm squire of cheesecloth with about 50 to 100 budworm eggs 0—24 hours old is also dipped in the test solution and placed on a leaf of the cotton plant, all being placed in the hood to dry. The leaf with the treated budworm eggs is removed from the plant and placed in a 236.6 ml (8-oz) dixie cup with a wet 5 cm piece of dental wick and covered with a lid. The other leaf is placed in a similar cup with a wick and a piece of cheesecloth infested with 50—100 newly hatched larvae is added before covering the cup with a lid. after 3 days at 26.7°C, 50% r.h., observations of egg hatch are made, as well as kill of newly hatched larvae, any inhibition of feeding, or interference of any sort with normal development.

Southern Armyworm — *Spodoptera eridania* (Cramer)

A Sieva lima bean plant with just the primary leaves expanded to 1.91 cm is dipped for 3 seconds with agitation in the "A" solution of 1000 ppm and set in the hood to dry. Following this, one leaf is placed in a 9 cm petri dish which contains a moist filter paper in the bottom and 10 third-instar armyworm larvae about 1 cm. long. This dish is covered and held at 26.7°C, and 50% RH. After 2 days, mortality counts and estimates of the amount of feeding are made.

*Secondary Tests*

Tobacco Budworm — *Heliothis virescens* (Fabricius) — Third Instar.

Three cotton plants with just expanded cotyledons are dipped in 1000 ppm solution and placed in the hood to dry. When dry, each cotyledon is cut in half, and each half is placed in one of ten 29.6 ml plastic medicine cups containing a 1.25 cm dental wick saturated with water and one third-instar budworm larva is added. The cup is capped and held for 3 days at 2 6.7°C, 50% r.h., after which mortality counts are made.

Cabbage Looper — *Trichoplusia ni* (Hübner) — Third Instar

A true leaf of a cotton plant is dipped into the test solution, agitated for 3 seconds, and removed to dry in an exhaust hood. When dry, the leaf is placed in 9.0 cm petri dish with moist filter paper on the bottom. Ten third-instar larvae are added and the lid placed on the dish. Mortality counts are made after 3 days at 26.7°C, and 50±10% r.h.

Imported Fire Ant Bait Toxicant Tests

*(Solenopsis invicta* Buren)

Test Procedure

Tests are conducted in 30 ml disposable plastic medicine cups (40 mm ID at the top, tapering to 32 mm ID at the bottom, 38 mm bigh). A hole (6 mm diam.) is drilled through the bottom of each cup and a layer of plaster of Paris and builders' cement (9:1 ratio) poured over the bottom. The plaster mixture covers the hole and acts as a wick to draw up water when the cup is placed on a 8 mm thick foam rubber pad saturated with water. The cups are placed in a tray and covered by another tray

inverted to prevent rapid evaporation of water from the foam pad. Moisture is necessary to keep the humidity in the cups high and thereby prevent disiccation of the ants. The cement is added to make a hard mixture through which the ants cannot tunnel and escape.

Twenty worker ants from field-collected colonies are placed in each test chamber *ca.* 24 hr preceding the start of the test. This pretreatment holding period allows time for recovery of the ants from handling and for orientation to the containers.

Candidate chemicals are dissolved directly in the food material; e.g. soybean oil. The toxic solution is offered to the ants on cotton swabs saturated with the material and placed in the test chamber in small vial caps. Preliminary tests are conducted at concentrations of 1.0, 0.1 and 0.01 percent.

The ants are allowed to feed as desired on the toxic bait for 24 hr. After this exposure period, the toxicant is removed from the chamber and the ants remain without food for an additional 24 hr. At the end of this time, new vial caps containing cotton swabs saturated with soybean oil are placed in the chamber and left for the remainder of the test period. Knockdown and mortality counts are made at 14 days following initial exposure. Each test consists of 3 replications. Room temperature is maintained at 23.9±1°C.

Data obtained are reported in the Table below.

TABLE

PERCENT MORTALITY

| Tobacco Budworm | | | | | | Third-Star | | | | | |
|---|---|---|---|---|---|---|---|---|---|---|---|
| Eggs | Larvae | | Southern Armyworms | | | Tobacco Budworms | | Cabbage Looper | | Imported Fire Ant | |
| 300 ppm | 300 ppm | 100 ppm | 1000 ppm | 100 ppm | 10 ppm | 1000 ppm | 100 ppm | 1000 ppm | 100 ppm | 10,000 ppm | 1000 ppm |
| 0 | 100 | 0 | 100 | 100 | 0 | 100 | 100 | 100 | 100 | 100 | 97 |

## Claims

1. The compound 1,5-bis[*p*-(trifluoromethoxyl)phenyl]-1,4-pentadien-3-one (1,4,5,6-tetrahydro-5,5-dimethyl-2-pyrimidinyl)hydrazone, or an inorganic acid addition salt thereof.

2. A method for controlling insects which comprises contacting said insects, their habitat (but not a human or animal body), and/or their food supply, with an insecticidally effective amount of a compound as defined in Claim 1.

3. A method according to Claim 2, wherein the insects are Lepidopterous insects, and the compound is applied at the rate of from 0.14 kg/hectare to 11.2 kg/hectare.

4. A method according to Claim 2, wherein the insects are ants, Family Formicidae, and the compound is applied at the rate of from 2.5 g/hectare to 37.5 g/hectare.

5. A method according to Claim 2, wherein the insects are termites, cockroaches, grasshoppers, flies and ants, Family Formicidae, and the compound is applied incorporated in a bait at a concentration of from 0.125% to 2.0% by weight.

6. A method according to Claim 5, wherein the ants are the southern fire ant *Solenopsis xyloni,* the black imported fire ant *Solenopsis richteri* and the red imported fire ant *Solenopsis invicta.*

7. A method for protecting agronomic crops, trees, shrubs and ornamentals from attack by insects which comprises applying to said crops an insecticidally effective amount of a compound as defined in Claim 1.

8. A method according to Claim 7, wherein said insects are Lepidopterous insects, and said compound is applied at the rate of from 0.28 kg/hectare to 11.2 kg./hectare.

9. A method according to Claim 7, wherein said insects are ants, Family Formicidae, and said compound is applied at the rate of from 2.5 g/hectare to 37.5 g/hectare.

10. A method according to Claim 7, wherein the insects are termites, cockroaches, grasshoppers, flies and ants, Family Formicidae, and the compound is applied incorporated in a bait at a concentration of from 0.125% to 2.0% by weight.

11. A method according to Claim 9, wherein the ants are the southern fire ant *Solenopsis xyloni,* the black imported fire ant *Solenopsis richteri* and the red imported fire ant *Solenopsis invicta.*

12. An insecticidal composition comprising a compound as defined in Claim 1 and a carrier or diluent therefor.

## Revendications

1. La(1,4,5,6-tétrahydro-5,5-diméthyl-2-pyrimidinyl)hydrazone, de 1,5-bis[p-trifluorométhoxy)-phényl]-1,4-pentadiène-3-one ou un sel d'addition avec un acide minéral de celle-ci.

2. Procédé pour lutter contre les insectes qui comprend la mise en contact desdits insectes, de leur habitat (mais non le corps de l'homme ou d'un animal) et/ou de leur apport alimentaire, avec une quantité insecticide efficace d'un composé tel que défini à la revendication 1.

3. Procédé selon la revendication 2, dans lequel les insectes sont des insectes lépidoptères et le composé est appliqué à la dose de 0,14 kg/ha à 11,2 kg/ha.

4. Procédé selon la revendication 2, dans lequel les insectes sont des fourmis de la famille des formicidés et le composé est appliqué à la dose de 2,5 g/ha à 37,5 g/ha.

5. Procédé selon la revendication 2, dans lequel les insectes dont des termites, des blattes, des sauterelles, des mouches et des fourmis de la famille des formicidés et le composé est appliqué incorporé à un appât à une concentration de 0,125% à 2,0% en poids.

6. Procédé selon la revendication 5, dans lequel les fourmis sont la fourmi de feu méridionale *Solenopsis xyloni,* la fourmi de feu noire importée *Solenopsis richteri* et la fourmi de feu rouge importée *Solenopsis invicta.*

7. Procédé pour protéger des cultures agronomiques, des arbres, des arbustes et des végétaux ornementaux contre l'attaque par des insectes qui comprend l'application auxdites cultures d'une quantité insecticide efficace d'un composé tel que définis à la revendication 1.

8. Procédé selon la revendication 7, dans lequel lesdits insectes sont des insectes lépidoptères et ledit composé est appliqué à la dose de 0,28 kg/ha à 11,2kg/ha.

9. Procédé selon la revendication 7, dans lequel lesdits insectes sont des fourmis de la famille des formicidés et ledit composé est appliqué à la dose de 2,5 g/ha à 37,5 g/ha.

10. Procédé selon la revendication 7, dans lequel les insectes sont des termites, des blattes, des sauterelles, des mouches et des fourmis de la famille des formicidés et le composé est appliqué, incorporé à un appât à une concentration de 0,125% à 2,0% en poids.

11. Procédé selon la revendication 9, dans lequel les fourmis sont la fourmi de feu méridionale *Solenopsis xyloni,* la fourmi de feu noire importée *Solenopsis richteri* et la fourmi de feu importée rouge *Solenopsis invicta.*

12. Composition insecticide comprenant un composé tel que défini à la revendication 1 et un véhicule ou diluant pour celui-ci.

**Patentansprüche**

1. Verbindung der Bezeichnung 1,5-Bis[p(trifluormethoxy)phenyl]-1,4-pentadien-3-on-(1,4,5,6-tetrahydro-5,5-dimethyl-2-pyrimidinyl)hydrazon oder ein anorganisches Säureadditionssalz derselben.

2. Verfahren zur Insektenbekämpfung durch Kontaktieren der Insekten oder deren Habitat (mit Ausnahme des menschlichen oder tierischen Körpers) und/oder deren Nahrungsquellen mit einer insektizidwirksamen Menge einer Verbindung gemäß Anspruch 1.

3. Verfahren nach Anspruch 2, dadurch gekennzeichnet, daß es sich bei den Insekten um Lepidopteren handelt und daß die Verbindung in einer Menge von 0,14 kg/ha bis 11,2 kg/ha angewendet wird.

4. Verfahren nach Anspruch 2, dadurch gekennzeichnet, daß es sich bei den Insekten um Ameisen der Familie Formicidae handelt und daß die Verbindung in einer Menge von 2,5 g/ha bis 37,5 angewendet wird.

5. Verfahren nach Anspruch 2, dadurch gekennzeichnet, daß es sich bei den Insekten um Termiten, Küchenschaben, Heuschrecken, Fliegen und Ameisen der Familie Formicidae handelt un daß die Verbindung in einem Köder mit einer Konzentration von 0,125 bis 2,0 Gew.-% einverleibt angewendet wird.

6. Verfahren nach Anspruch 5, dadurch gekennzeichnet, daß es sich bei den Ameisen um Solenopsis xyloni, Solenopsis richteri oder Solenopsis invicta handelt.

7. Verfahren zum Schutz von landwirtschaftlichen Ernten, Bäumen, Sträuchern und Zierpflanzen vor dem Angriff durch Insekten, dadurch gekennzeichnet, daß man eine insektizidwirksame Menge der Verbindung gemäß Anspruch 1 aufbringt.

8. Verfahren nach Anspruch 7, dadurch gekennzeichnet, daß die Insekten Lepidopteren sind und daß die Verbindung in einer Menge von 0,28 kg/ha bis 11,2 kg/ha angewendet wird.

9. Verfahren nach Anspruch 7, dadurch gekennzeichnet, daß es sich bei den Insekten um Ameisen der Familie Formicidae handelt und daß die Verbindung in einer Menge von 2,5 kg/ha bis 37,5/ha angewendet wird.

10. Verfahren nach Anspruch 7, dadurch gekennzeichnet, daß es sich bei den Insekten um Termiten, Küchenschaben, Heuschrecken, Fliegen und Ameisen der Familie Formicidae handelt und daß die Verbindung in einem Köder mit einer Konzentration von 0,125 bis 2,0 Gew.-% einverleibt angewendet wird.

11. Verfahren nach Anspruch 9, dadurch gekennzeichnet, daß es sich bei den Ameisen um Solenopsis xyloni, Solenopsis richteri oder Solenopsis invicta handelt.

12. Insektizides Mittel, gekennzeichnet durch einen Gehalt einer Verbindung gemäß Anspruch 1, sowie eines Trägerstoffs oder Verdünnungsstoffs dafür.